(19) Europäisches Patentamt
European Patent Office
Office européen des brevets

(11) Veröffentlichungsnummer: **0 171 072**
**A2**

(12) # EUROPÄISCHE PATENTANMELDUNG

(21) Anmeldenummer: **85109906.9**

(22) Anmeldetag: **06.08.85**

(51) Int. Cl.⁴: **C 12 Q 1/68**

(30) Priorität: **06.08.84 DD 265997**
**06.08.84 DD 265998**
**14.05.85 DD 276328**

(43) Veröffentlichungstag der Anmeldung: **12.02.86**
**Patentblatt 86/7**

(84) Benannte Vertragsstaaten: **AT BE CH DE FR GB IT LI NL SE**

(71) Anmelder: **Akademie der Wissenschaften der DDR, Otto-Nuschke-Strasse 22/23, DDR-1086 Berlin (DD)**

(72) Erfinder: **Rosenthal, André, Dr., Invalidenstrasse 135, DDR-1040 Berlin (DD)**
Erfinder: **Hunger, Hans-Dieter, Dr., Zelterstrasse 84, DDR-1297 Zepernick (DD)**
Erfinder: **Kagelmaker, Horst, Zelterstrasse 96, DDR-1297 Zepernick (DD)**
Erfinder: **Grätschus, Monika, Gärtnerstrasse 7, DDR-1035 Berlin (DD)**

(74) Vertreter: **Patentanwälte Beetz sen. - Beetz jun. Timpe - Siegfried - Schmitt-Fumian, Steinsdorfstrasse 10, D-8000 München 22 (DE)**

(54) **Verfahren und Vorrichtung zur Festphasen-Sequenzierung von Nucleinsäurefragmenten.**

(57) Die Erfindung betrifft ein Verfahren sowie eine Vorrichtung zur Festphasen-Sequenzierung von Nucleinsäurefragmenten, welche die gleichzeitige Sequenzierung grosser Stückzahlen von langen und kurzen Nucleinsäurefragmenten ermöglichen und automatisierbar sind.

Erfindungsgemäss wird ein mechanisch stabiler Flächenträger mit Anionenaustauscheigenschaften verwendet, an dem radioaktiv markierte Nucleinsäurefragmente immobilisiert werden. Das immobilisierte Fragment oder mehrere immobilisierte Fragmente werden gleichzeitig mit oder ohne Reagensüberschuss chemisch modifiziert und nach Waschen einzeln mit Piperidin umgesetzt, wobei gleichzeitig die Elution der Fragmente vom Träger erfolgt, die mit chemischen, ionischen oder elektromagnetischen Mitteln vorgenommen wird.

Das Verfahren und die Vorrichtung sind insbesondere in der Molekularbiologie und Gentechnik vorteilhaft anwendbar.

EP 0 171 072 A2

# Verfahren und Vorrichtung zur Festphasen-Sequenzierung von Nucleinsäurefragmenten

Die Erfindung betrifft ein Verfahren sowie eine Vorrichtung zur Sequenzanalyse von Nucleinsäurefragmenten (DNA und RNA) in der Molekularbiologie und Gentechnik bzw. zur Sequenzanalyse von Oligodesoxy- und Oligoribonucleotiden nach chemischer Synthese.

Es wurde bereits ein Festphasenverfahren zur Sequenzierung von DNA-Fragmenten angegeben (S. A. Chuvpilo und V. V. Kravchenko, Bioorg. Khim 9 (12) (1983) 1634 - 1637), das sich allerdings nur zur Sequenzierung langer DNA-Abschnitte eignet und bei dem ein kommerziell erhältliches DEAE-Papier Whatman DE 81 verwendet wird. Dieses Verfahren umfaßt folgende Schritte: 1) Immobilisierung des 5'- oder 3'-markierten DNA-Fragments am Träger; 2) Waschen; 3) chemische Modifizierungsreaktionen ohne Reagensüberschuß (d. h. nicht in einem Reaktionsgefäß) nach Maxam und Gilbert (A. M. Maxam und W. Gilbert, Methods in Enzymol. 65 (1980) 499 - 560); 4) Waschen; 5) Piperidin-Reaktion unter Standardbedingungen (nur 20 % Verlust der radioaktiven Fragmente); 6) Waschen; 7) 2 x Elution der Fragmente vom Träger mit 1 M NaCl bei 60 °C; 8) Ethanol-Präzipitation zur Ausfällung des Materials und 2 x Waschen mit 90 % Ethanol.

Dieses Verfahren hat folgende Nachteile:

- Die sehr schlechte mechanische Stabilität des DEAE-Papiers erschwert die Handhabung des Trägers in wäßrigen Puffern sehr (vor allem bei höheren Temperaturen). Es kommt zu schnellem Zerreißen, Auflösen und Abfleddern des Trägers.
- Die angewandten Modifizierungsreaktionen führen in der A+G-, T+C- und C-Reaktion zu nahezu 100 % Verlust, wenn sie in Reaktionsgefäßen (z. B. in Eppendorf-Hütchen) mit Reagensüberschuß ausgeführt werden.
- Aufgrund der schlechten mechanischen Eigenschaften des DEAE-Trägers ist die gleichzeitige Sequenzierung einer großen Anzahl von Nucleinsäurefragmenten in einem Reaktionsgefäß nicht möglich.
- Insgesamt sind acht Verfahrensschritte erforderlich, wobei die Ethanol-Präzipitation (Verfahrensschritt 8) prinzipiell nicht für eine automatische Durchführung geeignet ist.
- Oligonucleotide mit acht Einheiten können nach diesem Verfahren nicht sequenziert werden, da die ersten 5'-ständigen Nucleotide bei der Umfällung verlorengehen und damit die Sequenzierungsmuster nicht vollständig gelesen werden können.

Aufgrund der oben dargestellten Nachteile ist das beschriebene Verfahren zum einen nicht automatisierbar, zum anderen können selbst bei manueller Durchführung keine großen Anzahlen gleichzeitig sequenziert werden. Kurze Nucleinsäurefragmente (DNA- und RNA-Oligomere) können überhaupt nicht sequenziert werden.

Vorrichtungen zur Durchführung von Festphasensequenzierungen sind bisher noch nicht bekannt geworden. Das bekannte Sequenzierungsverfahren kann in der Regel in kommerziell erhältlichen Gefäßen, wie z. B. Eppendorf-Hütchen und üblichen Laborglasgeräten, durchgeführt werden. Die gleichzeitige Sequenzierung mehrerer Fragmente ist zwar prinzipiell möglich, jedoch ist der Ablauf des Verfahrens unökonomisch, und die Gefahr der Verwechslung von immobilisierten Nucleinsäurefragmenten ist groß.

Der Erfindung liegt die Aufgabe zugrunde, lange und kurze Nucleinsäurefragmente so an eine feste Phase zu binden, daß eine vollautomatische oder eine sichere manuelle Handhabung der Immobilisate möglich ist und die Nucleinsäurefragmente schnell, verwechslungsfrei und in großer Stückzahl simultan bestimmt werden können. Die Lösung der Aufgabe erfolgt erfindungsgemäß mit einem Festphasensequenzierungsverfahren für Nucleinsäurefragmente unter Verwendung eines neuartigen Flächenträgers mit Anionenaustauscheigenschaften und mit einer neuen Einrichtung zur Durchführung des Verfahrens.

Die Immobilisierung des oder der markierten Fragmente am Flächenträger erfolgt in an sich bekannter Weise, z. B. durch Auftropfen oder durch Elektroelution aus Gelen (pro Fragment 4 Träger). Diese Träger werden gewaschen, und anschließend werden das immobilisierte Fragment oder mehrere immobilisierte Fragmente gleichzeitig chemisch modifiziert. Die chemischen Modifizierungsreaktionen erfolgen mit bzw. ohne Reagensüberschuß. Ohne Reagensüberschuß werden die Flächenträger auf eine

chemisch inerte Matrix aufgelegt, worauf gerade so viel Reagentien mit Mikropipetten auf einen Flächenträger aufgetragen werden, daß er nur befeuchtet ist.

Nach einem Waschprozeß werden die sortierten Fragmente einzeln mit Piperidin umgesetzt und mit chemischen, ionischen oder elektromagnetischen Mitteln eluiert.

Als mechanisch stabiler Flächenträger mit Anionenaustauscheigenschaften wird ein Flächenträger verwendet, der durch Umsetzung bekannter geformter oder nicht geformter makromolekularer Massen mit Verbindungen der allgemeinen Formel I,

$$\left[ X - R - \underset{R^3}{\overset{R^1}{N}} - R^2 \right]^{\oplus} Y^{\ominus} \qquad (I),$$

entsteht, in der bedeuten:

X $NH_2$-, OH- oder SH-Gruppen

R Alkylgruppen mit 1 - 10 C-Atomen, aromatische Gruppen, photolytische Gruppen wie substituierte o-Nitrobenzylester bzw. substituierte Methylphenancylester oder Heterocyclen,

$R^1$, $R^2$, $R^3$ Alkylgruppen mit 1 - 4 C-Atomen oder H-Atomen
und

Y Halogenide oder Hydroxylgruppen.

Die für die Umsetzung geeigneten makromolekularen Massen haben folgende Zusammensetzung:

- 5 - 80 Vol.-% einer makromolekularen Verbindung,
- 1 - 80 Vol.-% einer makromolekularen Verbindung mit 4,6-Dihalogen-1,3,5-triazin-Gruppierungen,
- 0,5 - 50 Vol.-% 2,4,6-Trihalogen-1,3,5-triazin,
- bis 20 Vol.-% Halogenide,
- ggf. bis 5 % puffernde Substanzen
  sowie
- ggf. ein flüssiges Dispergiermittel.

Anstelle der direkten Umsetzung der geformten bzw. nicht geformten makromolekularen Massen mit chemisch aktiven Füllkörpern mit Verbindungen der allgemeinen Formel I können vorteilhafterweise die Verbindungen der Formel I intermediär durch Reaktion von Verbindungen der allgemeinen Formel II

$$\left[ Y - R - \overset{+}{N}H_3 \right]^{\oplus} Y^{\ominus} \qquad (II)$$

mit R und Y wie oben

mit tertiären Aminen der allgemeinen Formel III

$$\begin{array}{c} R^1 \\ | \\ N - R^2 \\ | \\ R^3 \end{array} \qquad (III)$$

erzeugt und entsprechend eingesetzt werden.

Im ersten Fall können geformte makromolekulare Massen mit chemisch aktiven Füllkörpern, wie z. B. mechanisch stabilen Cellulose-Papieren, Vliesen und Geweben, umgesetzt und auf diese Weise mechanisch stabile

Flächenträger mit Anionenaustauscheigenschaften und mit hoher Bindungskapazität für Nucleinsäuren von bis zu 120 µg/$cm^2$ erhalten werden.

Im zweiten Fall können nichtgeformte makromolekulare Massen mit chemisch aktiven Füllkörpern, wie z. B. sphärischer, kristalliner und faseriger Cellulose, umgesetzt und auf diese Weise Anionenaustauscher erhalten werden, die entweder in dieser Form eingesetzt oder mit flüssigen Dispergiermitteln zu geformten Flächenträgern mit Anionenaustauscheigenschaften verarbeitet werden.

Werden zur Umsetzung der geformten und nicht geformten makromolekularen Massen mit chemisch aktiven Füllmaterialien Verbindungen der allgemeinen Formel I bzw. Verbindungen der allgemeinen Formel II in Verbindung mit tertiären Aminen der Formel III eingesetzt, wobei R Alkylgruppen mit 1 - 10 C-Atomen oder aromatische Gruppen bedeutet, ergeben sich Anionenaustauscher, die chemisch mit primären, sekundären und tertiären Aminen eluierbar sind. Diese chemische Elution hat besondere Bedeutung für das erfidnungsgemäße Festphasen-Sequenzierungsverfahren von Nucleinsäurefragmenten bzw. zur Isolierung von DNA-Fragmenten aus Agarose- oder Polyacrylamid-Gelen.

Werden zur Umsetzung der geformten und nicht geformten makromolekularen Massen mit chemisch aktiven Füllkörpern Verbindungen der allgemeinen Formel I eingesetzt, wobei R substituierte o-Nitrobenzyl- oder substituierte Methylphenancylester bedeutet, ergeben sich Anionenaustauscher, die

photolytisch, d. h. unter Verwendung elektromagnetischer Strahlung, eluierbar sind, was einen Vorteil hinsichtlich der Automatisierbarkeit des Verfahrens bedeutet. Alle obengenannten Anionenaustauscher sind zusätzlich mit ionischen Mitteln, d. h. mit Lösungen hoher Ionenstärke von Salzen, wie z. B. $NH_4HCO_3$, $(NH_4)_2CO_3$, $Et_3NHCO_3$, $Et_3NHAc$, $NH_4Ac$, $K_2HPO_4$, NaCl u.a., eluierbar.

Die beschriebenen Anionenaustauscher besitzen eine hohe Bindungskapazität und weisen insbesondere in der Flächenform eine hervorragende mechanische Stabilität auf. Aus diesem Grund ist der Einsatz der geformten Flächenträger mit Anionenaustauscheigenschaften auch in der weiteren molekularbiologischen und biochemischen Forschung besonders angezeigt. Gegenüber herkömmlichen Anionenaustauschern sind die erfindungsgemäßen Flächenträger neben der ionischen Eluierbarkeit auch mit chemischen Mitteln oder mit elektromagnetischer Strahlung eluierbar.

Zur Immobilisierung werden 1 oder n 5'- oder 3'-markierte lange oder kurze Nucleinsäurefragmente auf 4 oder 4 x n einzelnen mechanisch stabilen Flächenträgern (ca. 1 bis 2 x 1 bis 2 mm) mit Anionenaustauscheigenschaften aufgetragen.

G- A+G- T+C- C-Reaktion

ein Fragment

□ □ □ □

fünf Fragmente

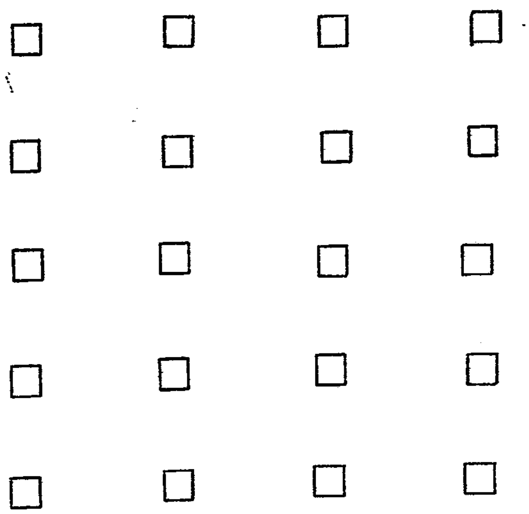

Alternativ werden n 5'- oder 3'-markierte lange oder kurze Nücleinsäurefragmente jeweils auf 4 einzelnen, aber geometrisch größeren Flächenträgern immobilisiert, so daß alle n Fragmente mit einem Sicherheitsabstand jeweils auf einem Flächenträger fixiert sind.

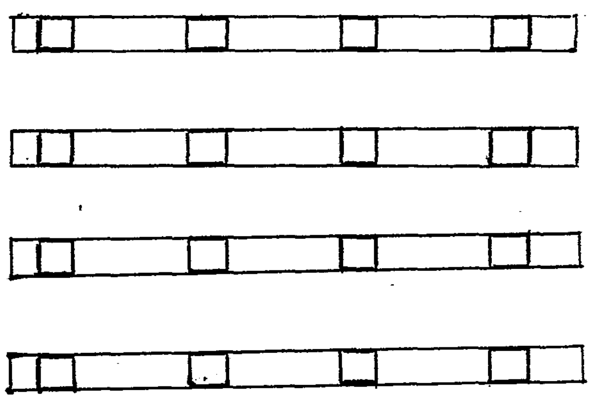

G-Reaktion

A+G-Reaktion

T+C-Reaktion

C-Reaktion.

Alle Flächenträger müssen speziell gekennzeichnet sein. Das nachfolgende Waschen der Flächenträger erfolgt 2x nacheinander in Wasser und in Ethanol, wobei sie von Salzen oder anderen Verunreinigungen befreit werden.

Danach werden sie kurz an der Luft oder gegebenenfalls mit erwärmter Luft getrocknet.

Zur chemischen Modifizierung wird wie folgt verfahren:

a) 1 Fragment auf vier einzelnen Flächenträgern wird mindestens vier verschiedenen chemischen Modifizierungsreaktionen (G, A+G, T+C, C) unterworfen. Jeweils ein Flächenträger (1 bis 2 mm x 1 bis 2 mm) wird dazu in ein spezielles Reaktionsgefäß, z. B. ein Eppendorf-Hütchen, einen Glasreaktor mit automatischer Zu- und Ablaufregulierung (Automat) gebracht. Der Flächenträger wird dann mit Reagensüberschuß behandelt, d. h. er schwimmt während der Reaktion im Reaktionsmedium. Folgende Modifizierungsreaktionen können angewandt werden: G-Reaktion mit DMS; A+G-Reaktion mit Ameisensäure, Piperidinformiat pH 2 oder mit Diethylpyrocarbonat; T+C-Reaktion mit Kaliumpermanganat oder Osmiumtetroxid; C-Reaktion mit Hydroxylamin. Nach Beendigung der Reaktion wird der Flächenträger manuell, z. B. mit einer Pinzette, aus dem Reaktionsgefäß entfernt, bzw. der Reagensüberschuß wird im automatischen System durch Ablassen entfernt.

b) n Fragmente, die auf 4 x n Flächenträgern aufgetragen (immobilisiert) sind, werden in G-, A+G-, T+C- und C-Fragmente sortiert. Jeweils die n-G, n-A+G, n-T+C und n-C-Fragmente werden in vier Reaktionsgefäßen nach a) gleichzeitig mit Reagensüberschuß der jeweiligen Modifizierungsreaktion unterworfen.

c) 1 Fragment auf vier einzelnen Flächenträgern oder n Fragmente auf 4 x n einzelnen Flächenträgern werden nicht in Reaktionsgefäßen, sondern auf chemisch inerten Unterlagen wie Glasplatten oder Kunststofffolien einzeln aufgebracht und ohne Reagensüberschuß den vier einzelnen Modifizierungsreaktionen unterworfen. Dazu werden gerade so viel Reagentien mit Mikropipetten auf einen Flächenträger aufgetragen, daß er nur befeuchtet ist (für 1 bis 2 mm x 1 bis 2 mm eines Cellulosepapiers mit Anionenaustauscheigenschaften ca. 1 - 5 µl Reagens).

d) n Fragmente werden nicht auf 4 x n (1 bis 2 $mm^2$) große Flächenträger aufgebracht, sondern auf vier geometrisch größere Flächenträger derart aufgetragen, daß alle n Fragmente mit einem Sicherheitsabstand jeweils auf einem Flächenträger fixiert sind. Nach a) oder c) werden mit oder ohne Reagensüberschuß diese Flächenträger den Modifizierungsreaktionen unterworfen. Nach a) sind dabei vier den geometrisch größeren Flächenträgern angepaßte Reaktionskammern nötig. Nach c) sind entsprechend größere Volumina zur Befeuchtung zu wählen.

Nach Beendigung der Modifizierungsreaktionen werden die chemisch modifizierten Flächenträger zweimal nacheinander mit Wasser und Ethanol sorgfältig gewaschen. Das geschieht manuell, indem die Flächenträger mit der Pinzette in die entsprechenden Lösungsmittel getaucht, jeweils zwischen Fließpapier gelegt und unter Andrücken getrocknet werden. Die sich nach c) in größerer Anzahl ergebenden Flächenträger können gemeinsam gewaschen, müssen aber danach wieder auseinandersortiert werden. In automatischen Systemen wird nach Ablassen der Modifizierungsreagentien die

Träger in den Reaktoren mit Fritte ebenfalls durch kontinuierliche oder diskontinuierliche Zudosierung von Wasser und Ethanol gewaschen. Anschließend werden alle Flächenträger mit nur einem immobilisierten Fragment einzeln mit einer frischen 10-%igen wäßrigen Piperidinlösung 15 bis 45 min bei 90 °C behandelt. Dabei kommt es zum DNA-Strangbruch, und gleichzeitig erfolgt die Elution vom Träger. Die geometrisch größeren Flächenträger mit n immobilisierten Fragmenten werden so zerschnitten, daß aus einem größeren Träger n einzelne Stücke entstehen. Diese werden wie oben einzeln mit Piperidin behandelt.

Zur Elution der Fragmente vom Träger werden chemische, ionische oder elektromagnetische Mittel verwendet. Zur ionischen Elution verwendet man Lösungen hoher Ionenstärke von Salzen wie $(NH_4)HCO_3$, $(NH_4)_2CO_3$, $Et_3NHCO_3$, $Et_3NHAc$, $NH_4Ac$, $K_2HPO_4$ u.a.

Das erfindungsgemäße Verfahren hat folgende Vorteile:

1. Während sämtlicher Manipulationen ist eine hervorragende mechanische Stabilität gewährleistet. Diese mechanische Stabilität, verbunden mit der chemischen Elution während der Piperidin-Reaktion, ermöglicht die vollständige Automatisierbarkeit, da keine Fällungsschritte im Gesamtprozeß durchzuführen sind.

2. Die mechanische Stabilität der Flächenträger eröffnet die Möglichkeit, bei Reagensüberschuß, d. h. im Reaktionsgefäß, eine große Anzahl von

Nucleinsäurefragmenten gleichzeitig zu sequenzieren.

3. Aufgrund der chemischen Elution während der Piperidin-Reaktion kann eine ionische Elution vermieden werden, die Fällungsreaktionen notwendig macht und dadurch den Verlust kurzer Fragmente bewirkt. Kurze DNA/RNA-Oligomere lassen sich vorteilhaft sequenzieren.

4. Es ist sowohl die manuelle als auch die automatische Sequenzierung großer Stückzahlen von kurzen und langen Nucleinsäurefragmenten möglich.

Im folgenden wird die erfindungsgemäße Vorrichtung beschrieben, mit deren Hilfe eine gleichzeitige Immobilisierung einer großen Anzahl von Nucleinsäurefragmenten auf einem Flächenträger, eine gleichzeitige chemische Weiterbehandlung, gleichzeitiges Ausstanzen der Festphasenimmobilisate, eine weitere gleichzeitige chemische Behandlung sowie gleichzeitige Nachbehandlungen wie Waschen, Hoch- und Tieftemperaturbehandlung und Zentrifugieren möglich sind.

Die Vorrichtung weist erfindungsgemäß Sequenzierungsblöcke mit dazugehörigen Deckeln, Flächenträgerhalter, Probendosierer und Stanzer auf. Sowohl Deckel und Flächenträgerhalter als auch Probendosierer und Stanzer sind durch Anbringung von Markierungen und Führungen nur in einer Stellung mit dem Sequenzierungsblock kombinierbar, so daß Vertauschungen der Proben ausgeschlossen sind. Der Sequenzierungsblock besteht aus einem quader- oder würfelförmigen Grundblock, wobei auch andere Formen möglich sind, in den Reaktionsgefäße eingelassen oder eingearbeitet sind. Die Anzahl der Reaktionsgefäße richtet sich

nach dem Umfang der zu analysierenden Einzelproben.

Eine Markierung gibt die Konstellation der Zuordnung der weiteren Teile der Vorrichtung an, die aufgrund des Verfahrensablaufs erfindungsgemäß benötigt werden.

Der Deckel ist so ausgebildet, daß er genau auf den Sequenzierungsblock paßt und dabei jedes eingelassene Reaktionsgefäß dicht verschließt. Er besteht aus einer nicht deformierbaren starren Abdeckung, an der eine elastische Dichtung befestigt ist, die auf die Reaktionsgefäße drückt, und Befestigungselementen zur Fixierung am Sequenzierungsblock.

Weiterhin gehört zur erfindungsgemäßen Vorrichtung ein Probendosierer für das gleichzeitige Auftragen der flüssigen Proben auf den Flächenträger. Der Probendosierer besitzt entsprechend der Anzahl der im Sequenzierungsblock vorhandenen Reaktionsgefäße gleich lange Kapillaren, die in einem Kapillarhalter stecken und das gleiche Verteilungsmuster wie die Reaktionsgefäße aufweisen, wobei also jede Kapillare einem anderen Reaktionsgefäß zugeordnet ist. Auch hier wird durch Markierungen und Führungen erreicht, daß die Kapillaren immer nur demselben Probengefäß zugeordnet sind. Der erfindungsgemäß eingesetzte Stanzer, mit dessen Hilfe die auf dem Flächenträger befindlichen Probenimmobilisate ausgestanzt und gleichzeitig in die Reaktionsgefäße des Sequenzierungsblocks überführt werden, besteht aus einer Platte mit eingesetzten Stanzstiften, die das gleiche Verteilungsmuster wie die Reaktionsgefäße im Sequenzierungsblock aufweisen. Die Probendosierung

und das Stanzen erfolgen nach dem Einlegen des Flächenträgers in einen Flächenträgerhalter, der auf dem Sequenzierungsblock in vorgeschriebener eindeutiger Stellung einrastet. Die Probendosierung kann allerdings auch außerhalb des Sequenzierungsblocks erfolgen.

Zu einer Grundausstattung der Vorrichtung gehören zweckmäßigerweise fünf Sequenzierungsblöcke, vier Deckel, vier Flächenträgerhalter, ein Probendosierer und ein Stanzer. Bei der Durchführung des Sequenzierungsverfahrens erfolgen alle wesentlichen Operationen mit oder im Sequenzierungsblock. Das ist ein bemerkenswerter Vorteil, der ein ökonomisches Arbeiten ermöglicht und Verwechslungen ausschließt.

Die Erfindung wird im folgenden anhand von Ausführungsbeispielen und der Zeichnungen näher erläutert; es zeigen:

Fig. 1a: Einen Sequenzierungsblock in halbgeschnittener Draufsicht mit Deckel,

Fig. 1b: den Sequenzierungsblock von Fig. 1a in Schnittdarstellung mit Deckel,

Fig. 1c: den Sequenzierungsblock von Fig. 1a mit tiefgezogenen Reaktionsgefäßen,

Fig. 2a: die Probenahme,

Fig. 2b: die Probendosierung,

Fig. 3 : einen Sequenzierungsblock mit Stanzvorrichtung
und

Fig. 4: ein Ablaufschema.

Beispiel 1

Cellulose-Papier, z.B. Whatman 540-Papier, wird nach EP 134 025 mit Cyanurchlorid aktiviert und sofort verpackt. Das auf diese Weise oberflächenbehandelte Papier (5 x 7 cm) wird zur Verwendung aus seiner speziellen Verpackung (geschweißte Folie, die unter $N_2$-Begasung bei -20 °C gelagert wird) entnommen und in eine gläserne Reaktionskammer gelegt, in der 2 mmol (450 mg) Triethyl-1-aminoethyl-ammoniumbromid in 7 ml Acetonitril vorgelegt wurden. Die Reaktionskammer wird dann ca. 12 h bei Raumtemperatur geschüttelt. Nach Beendigung der Reaktion wird das Papier mehrmals mit Wasser gewaschen und zwischen Fließpapier getrocknet. Anstelle des Lösungsmittels Acetonitril können auch wäßrige Puffersysteme verwendet werden.

Beispiel 2

400 mg kristalline, underivatisierte Cellulose wird nach EP 134 025 mit Cyanurchlorid aktiviert und anschließend in einem 25-ml-Rundkolben mit 2 mmol (450 mg) Triethyl-1-aminoethyl-ammoniumbromid in 7 ml Acetonitril 12 h bei Raumtemperatur umgesetzt. Nach Beendigung der Reaktion wird die Cellulose auf einer Fritte mehrmals mit Wasser gewaschen und wie in Beispiel 1 getrocknet.

Beispiel 3

Cellulose-Papier (Whatman 540-Papier) wird wie in Beispiel 1 mit Cyanurchlorid oberflächenaktiviert und anschließend in eine gläserne Reaktionskammer gelegt, in der 2 mmol (410 mg) 1-Brom-2-aminoethan-hydrobromid in 7 ml Acetonitril vorgelegt wurden. Die Reaktion wird sodann unter Zugabe von 5 ml Triethylamin gestartet. Die Reaktionskammer wird mit Parafilm abgedichtet und ca. 12 h bei Raumtemperatur geschüttelt. Nach Beendigung der Reaktion wird das Papier wie in Beispiel 1 gewaschen und getrocknet.

Beispiel 4

400 mg kristalline, underivatisierte Cellulose (z.B. Whatmann-Cellulose) wird wie in Beispiel 2 mit Cyanurchlorid aktiviert und anschließend in einem 25-ml-Rundkolben mit 2 mmol (410 mg) 1-Brom-2-aminoethan-hydrobromid in 7 ml Acetonitril versetzt. Die Reaktion wird durch Zugabe von 5 ml Triethylamin gestartet und nach 12 h bei Raumtemperatur durch Waschen der Cellulose und Trocknen, wie in Beispiel 2 beschrieben, beendet.

Beispiel 5

Oberflächenaktiviertes Cellulose-Papier (Whatman 540-Papier, 5 x 7 cm) von Beispiel 1 wird in einer Reaktionskammer mit 2 mmol (574 mg) p-Aminobenzyltriethylammoniumbromid in 7 ml Acetonitril 12 h bei Raumtemperatur umgesetzt und wie in Beispiel 1 weiter aufgearbeitet.

Beispiel 6

400 mg kristalline, nach Beispiel 2 oberflächenaktivierte Cellulose (Whatman-Cellulose) wird mit 2 mmol (574 mg) p-Aminobenzyl-triethylammoniumbromid in 7 ml Acetonitril, wie in Beispiel 2 beschrieben, umgesetzt und aufgearbeitet.

Beispiel 7

Oberflächenaktiviertes Cellulose-Papier (Whatman 540-Papier, 5 x 7 cm) von Beispiel 1 wird in einem Reaktionsgefäß mit 2 mmol (540 mg) p-Aminobenzylbromid-hydrobromid in 7 ml Acetonitril versetzt, worauf die Reaktion mit 5 ml Triethylamin gestartet wird. Nach 12 h Umsetzung bei Raumtemperatur wird das Papier wie in Beispiel 1 gewaschen und getrocknet.

Beispiel 8

400 mg kristalline, nach Beispiel 2 oberflächenaktivierte Cellulose (Whatman-Cellulose) wird analog Beispiel 4 mit 2 mmol (540 mg) p-Aminobenzylbromidhydrobromid in Acetonitril und mit Triethylamin umgesetzt und nach Beendigung der Reaktion gewaschen und getrocknet.

Beispiel 9

Oberflächenaktiviertes Cellulose-Papier (Whatman 540-Papier, 5 x 7 mm) von Beispiel 1 wird mit 2 mmol Glycin-(o-nitro-p-methyl—triethylammonium-

bromid)-benzylester in 7 ml Acetonitril 12 h bei Raumtemperatur in einer Reaktionskammer umgesetzt und wie in Beispiel 1 weiter aufgearbeitet.

Beispiel 10

Halbsynthetisches Papier auf der Basis von Polyamid und Cellulose (Hekosyn® ) wird wie in Beispiel 1 mit Cyanurchlorid oberflächenaktiviert und anschließend in eine gläserne Reaktionskammer gelegt, in der 2 mmol (410 mg) 1-Brom-2-aminoethan-hydrobromid in 7 ml Acetonitril vorgelegt wurden. Die Reaktion wird sodann unter Zugabe von 5 ml Triethylamin gestartet. Die Reaktionskammer wird mit Parafilm abgedichtet und ca. 12 h bei Raumtemperatur geschüttelt. Nach Beendigung der Reaktion wird das Papier wie in Beispiel 1 gewaschen und getrocknet.

Beispiel 11

Manuelle Sequenzierung einzelner Oligonucleotide mit Kettenlängen von 4 bis 15 Einheiten

1. Radioaktive Markierung der DNA-Sequenzen

10 - 50 pmol Oligonucleotid (oder längeres Fragment) werden nach üblichen Methoden am 5'-Ende mit $\gamma^{32}$P-ATP und Polynucleotid-Kinase markiert. Nach Beendigung der Kinase-Reaktion wird das Gesamtvolumen auf ein z.B. 20 %iges Polyacrylamid-Gel, das gegebenenfalls Harnstoff (7 M) enthält, aufgetragen und gelelektrophoretisch von ATP und anderen Verunreinigungen wie nicht er-

wünschten Nebenprodukten abgetrennt. Die gewünschte DNA-Bande wird durch kurze Autoradiographie auf Röntgenfilm sichtbar gemacht und aus dem Gel herausgeschnitten. Das Gelstück wird zerkleinert und in ein Eppendorf-Hütchen übergeführt. Das markierte Oligonucleotid wird durch zweimalige Elution mit Wasser bei einer Temperatur zwischen 37 und 60 °C während 30 min wiedergewonnen.

2. Immobilisierung der DNA-Sequenzen auf den Flächenträger mit Anionenaustauscheigenschaften

1 - 2 µl der unter 1. erhaltenen wäßrigen Lösung, die neben dem markierten Oligonucleotid gegebenenfalls Salze und Harnstoff enthält, werden auf mindestens vier ca. 2 x 2 mm große Stücke des Flächenträgers (Beispiele 1 - 10) aufgetropft. Nach kurzer Trocknung der Papierstückchen bei Raumtemperatur oder mit erwärmter Luft wird das Auftropfen so lange wiederholt, bis ca. 10.000 bis 50.000 cpm pro Flächenträger an einem Scintillationsmeßgerät gemessen werden. Die Papiere werden dann mit einer Pinzette zweimal nacheinander in Wasser und Ethanol gewaschen (ca. 1 min) und nach jeder Waschoperation zwischen Fließpapier kurz unter Druck getrocknet.

3. Chemische Modifizierungsreaktionen für die Oligonucleotide d(TCTA), d(TCTAGA), d(GTGAAUUCAC), d(TTCTTCTACACACCC) und d(TGATCAGATGGCTTT) mit Reagensüberschuß im Reaktionsgefäß

Die nach 2. von jedem Oligonucleotid erhaltenen

vier Papierstückchen mit immobilisierten markierten Fragment werden jeweils einzeln in ein Eppendorf-Hütchen gebracht. Die Eppendorf-Hütchen sind entsprechend der verwendeten Modifizierungsreaktion gekennzeichnet. Insgesamt liegen nunmehr von den fünf Oligonucleotiden 5 x 4 = 20 Eppendorf-Hütchen vor. Folgende Reagentien werden zupipettiert:

G-Reaktion:
- 200 µl Kakodylat-Puffer pH 8 oder 200 µl Ammoniumformiat-Puffer pH 3,5
- 1 bis 2 µl DMS;

A+G-Reaktion: 80 µl 88-%ige Ameisensäure;

T>C-Reaktion: 80 µl einer $10^{-4}$ M $KMnO_4$-Lösung, die kurz zuvor aus einer $10^{-2}$ M Stammlösung hergestellt wurde;

C-Reaktion: 40 µl einer 4 M Hydroxylamin-Lösung pH 6, die aus Hydroxylamin-hydrochlorid mit Triethylamin hergestellt wurde.

Die Reaktionszeiten betragen für die G-Reaktion 10 min und für alle anderen Reaktionen 20 min. Außer den erwähnten Reaktionen können auch die T>C-Reaktion mit Osmiumtetroxid (80 µl einer 5 mM $OsO_4$-Lösung + 1 µl Pyridin, 15 min bei 0 °C), die A+G-Reaktion mit Piperidinformiat (80 µl Piperidinformiat pH 2, 1 h bei 37 °C) oder Diethylpyrocarbonat (150 µl A-Puffer (50 mM Natriumacetat pH 5 + 1 mM EDTA) + 5 bis 10 µl einer frisch hergestellten 10 % DEPC-Lösung in Ethanol, 20 min bei 90 °C) durchgeführt werden. Die T+C-und C-Reaktionen mit Hydrazin nach Maxam und Gilbert bzw die A>C-Reaktion mit 1,2 M NaOH-Lösung führen zum vollständigen Verlust der Radioaktivität und können deshalb nicht verwendet

werden. Während der o. g. Modifizierungsreaktionen treten folgende Verluste auf: 20 % in der G-, 50 % in der A+G-, 0 % in der T > C- und 50 - 80 % in der C-Reaktion. Diese werden durch doppelte oder vierfache Radioaktivität in der A+G- bzw. in der C-Reaktion ausgeglichen. Die Modifizierungsreaktionen werden dadurch beendet, daß die Papiere mit einer Pinzette aus den Reaktionsgefäßen entfernt und nacheinander zweimal mit Wasser und Ethanol gewaschen werden.

4. Piperidin-Reaktion zur Erzielung des DNA-Strangbruchs und zur gleichzeitigen Elution der Fragmente vom Träger

Die 20 Papier-Träger werden einzeln in 20 neue Eppendorf-Hütchen gebracht. Nach Zugabe von 50 µl einer 10-%igen wäßrigen Piperidinlösung werden sie 30 min auf 90 °C gehalten. Nach Beendigung der Reaktion werden die Papiere mit einer Pinzette aus den Eppendorf-Hütchen entfernt, die Lösungen 1 min bei -200 °C, z. B. in flüssiger Luft, aufbewahrt und im Vakuum lyophilisiert (ca. 1 h). Der Lyophilisationsschritt wird zweimal mit 10 bis 20 µl Wasser wiederholt (jeweils ca. 30 min). Die Proben sind dann zum Auftrag für die Gelelektrophorese bereit.

Beispiele von Autoradiogrammen für o. g. Oligonucleotide:

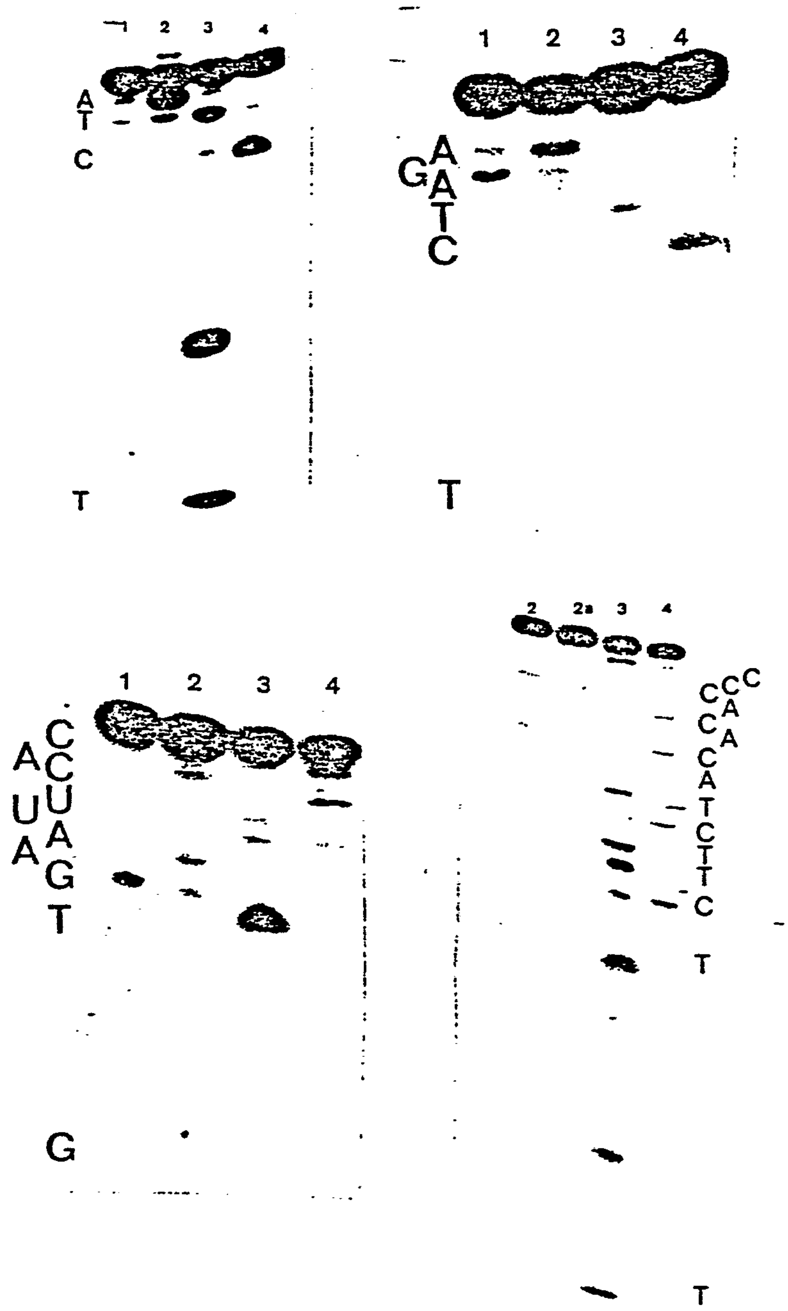


## Beispiel 12

**Manuelle gleichzeitige Sequenzierung großer Stückzahlen von Oligonucleotiden mit Reagensüberschuß im Reaktionsgefäß**

Markierung und Immobilisierung von fünf Pentadecanucleotiden d(TTCTTCTACACACCC), d(TGATCAGATGGCTTT), d(CTCCTGGCCATTCCT), d(GGGTACCCAGAAGTC) und d(TCGCTGAGATCACCA) erfolgen wie in Beispiel 11 (1. und 2.) beschrieben. Die chemischen Modifizierungsreaktionen werden nunmehr nur in vier Eppendorf-Hütchen durchgeführt, wobei in jedem jeweils fünf Papier-Träger enthalten sind. Die Reaktionsbedingungen und die nachfolgenden Waschoperationen sind wie in Beispiel 11 (3.). Nach Waschen und Trocknen der Papier werden die vorher gekennzeichneten Träger wieder auseinandersortiert, so daß jeweils wieder vier einzelne Papiere pro Oligonucleotid vorliegen und für die nachfolgenden Piperidin-Reaktion jeweils einzeln in ein Eppendorf-Hütchen gebracht werden können. Die Piperidin-Reaktion erfolgte analog Beispiel 11 (4.).

Beispiele von Autoradiogrammen für o. g. Oligonucleotide:

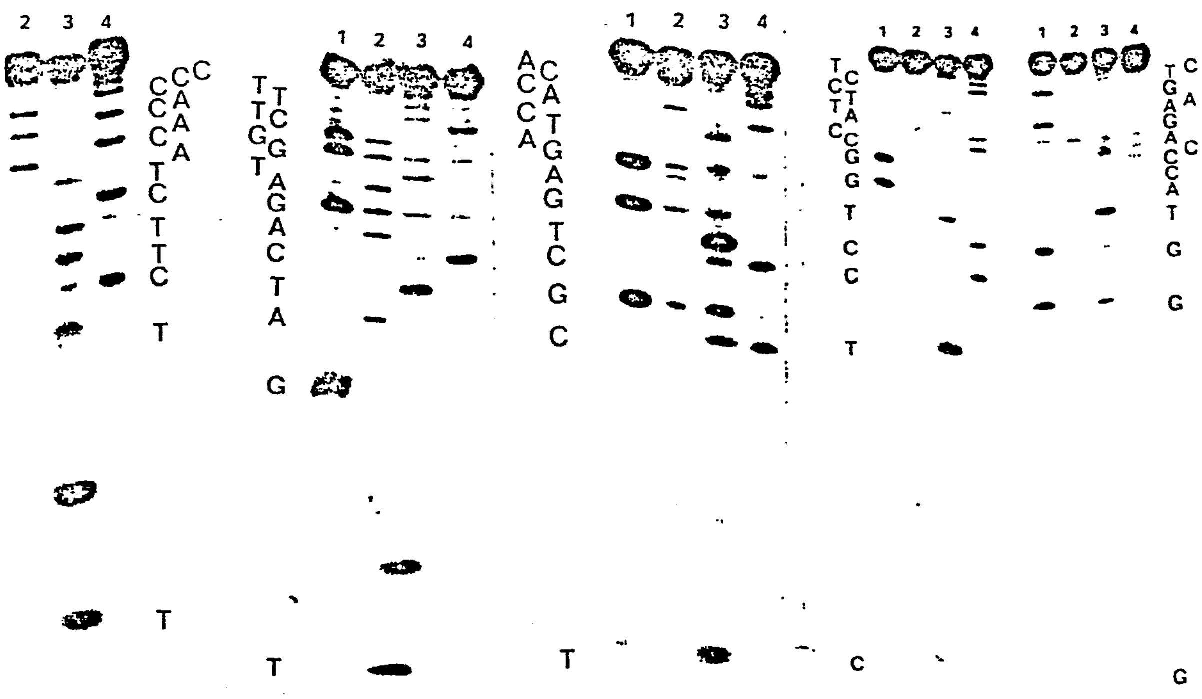

## Beispiel 13

### Manuelle gleichzeitige Sequenzierung großer Stückzahlen von Oligonucleotiden mit Reagensüberschuß in Reaktionsgefäßen

Die Markierung der fünf Pentadecanucleotide von Beispiel 12 erfolgt wie in Beispiel 11 (1.) beschrieben. Die Immobilisierung der Fragmente erfolgt diesmal nicht auf 4 x n (n = 5), also 20 (2 x 2 mm großen) Stücken des Flächenträgers, sondern auf 4 (2 x 20 mm großen) Stücken dadurch, daß ca. 1 - 2 µl jedes Oligonucleotids auf eine Fläche von 2 x 2 mm aufgetropft wird, und insgesamt alle fünf Oligonucleotide auf den Gesamt-Flächenträger aufgebracht werden. Die vier größeren Flächenträger werden in größeren Reaktionsgefäßen (entsprechend der Größe der Flächenträger) den bereits beschriebenen Modifizierungsreaktionen unterworfen. Nach Waschen und Trocknen der Träger werden die vier (2 x 20 mm großen) Flächenträger mit Hilfe einer Schere in jeweils fünf kleinere (2 x 2 mm große) Papierstücke zerschnitten und für die Piperidin-Reaktion in insgesamt 20 Eppendorf-Hütchen einzeln eingebracht. Die Piperidin-Reaktion sowie die Lyophilisierung erfolgen wie oben beschrieben.

## Beispiel 14

### Manuelle Sequenzierung von langen DNA-Sequenzen mit Reagensüberschuß in Reaktionsgefäßen

Lange DNA-Sequenzen werden völlig analog, wie in den Beispielen 11, 12 und 13 beschrieben, sequenziert. Lediglich die Reaktionszeiten der unter Beispiel 11 (3.) aufgelisteten Modifizierungsreaktionen müssen auf folgende Werte verkürzt werden:
G- 3: bis 5 min, alle anderen Reaktionen 10 min.
Die Verluste an Radioaktivität sind bei den langen DNA-Fragmenten während der Modifizierungsreaktionen wesentlich geringer.

Beispiel 15

Manuelle Sequenzierung von DNA-Sequenzen ohne Reagensüberschuß

Markierung und Immobilisierung der DNA-Sequenzen erfolgen analog Beispiel 11 (1. und 2.) und 13.
Die Flächenträger werden nunmehr jedoch nicht in Reaktionsgefäßen, wie Eppendorf-Hütchen, sondern auf eine ebene, mit einer Plastikfolie bedeckte Unterlage gebracht und nur mit solchen Volumina der Modifizierungsreagentien (Beispiel 11 (3.)) versetzt, daß sie gerade durchfeuchtet sind. In der Regel betragen die Volumina für 2 x 2 mm große Papiere ca. 1 - 3 µl. Werden größere Flächenträger, z. B. 2 x 20 mm, verwendet, muß das Volumen der Reagentien etwas erhöht werden. Alle anderen Operationen, wie die Modifizierungsreaktionen und ihre Dauer, das Waschen und die Piperidin-Reaktion, werden wie oben beschrieben durchgeführt.

Beispiel 16

Automatische Durchführung des erfindungsgemäßen Verfahrens

Ein erfindungsgemäßer DNA/RNA-Sequenzierungsautomat ist wie folgt aufgebaut: Im einfachsten Fall zur Sequenzierung nur eines Nucleinsäurefragments werden vier mit einer Fritte ausgerüstete, thermostatierbare Reaktoren mit einem Fassungsvermögen von maximal 250 µl mit dosierbarem Zu- und Ablauf parallel angeordnet. Die vier einzelnen Flächenträger (2 x 2 mm) mit den immobilisierten markierten Nucleinsäurefragmenten werden in die Reaktoren eingebracht. Nun erfolgt nach einem vorgegebenen Programm der automatische Ablauf der Operationen: 1) Waschen; 2) Chemische Modifizierungsreaktionen; 3) Waschen; 4) Piperidin-Reaktion (Elution). Dazu werden die individuellen Reagentien nach Beispiel 11 (3.) über Dosierpumpen hinzugegeben und verbleiben für die festgelegte Zeit bei geschlossenem Ablaufventil im Reaktor. Nach Beendigung der Reaktionen werden die Ventile der Reaktoren geöffnet, und die Reagentien fließen gegebenenfalls unter leichtem Luftdruck ab. Alle Waschschritte mit Wasser und Ethanol können kontinuierlich (bei geöffnetem Ablaufventil werden in die Reaktoren abwechselnd die o. g. Lösungsmittel geleitet) oder diskontinuierlich erfolgen (100 µl der o. g. Lösungsmittel werden bei geschlossenem Ablaufventil in den Reaktor geleitet und verbleiben dort für kurze Zeit, bevor sie wieder abgelassen werden). Bei dieskontinuierlichem Waschen bzw. am Ende des kontinuierlichen Waschprozesses können die Träger gegebenenfalls für wenige Sekunden mit erwärmter Luft getrocknet werden. Zur Durchführung der Piperidin-Reaktion werden ca. 25 - 50 µl 10-%ige wäßrige Piperidinlösung in die vier Reaktoren gepumpt (bei geschlossenem Ablaufventil) und der (die) Reaktoren (30 min) auf 90 °C erwärmt. Nach

Öffnen der Ablaufventile werden die Piperidinvolumina unter leichtem Luftüberdruck in vier einzelne Eppendorf-Hütchen geblasen, die über PTFE-Schläuche (Ø 0,4 - 1 mm) mit den Reaktoren verbunden sind. Die vier Eppendorf-Hütchen mit den eluierten Fragmenten werden entweder direkt im Automaten innerhalb einer speziellen Vakuumkammer lyophilisiert, oder die Lyophilisation wird außerhalb des Systems vorgenommen. Im ersten Fall wird nach der ersten Lyophilisation die Prozedur mit jeweils 10 bis 20 µl Wasser, die automatisch zugeführt werden, wiederholt (zwei- bis dreimal). Die Gesamtzeit des Operationszyklus beträgt ca. 2,5 h. Im zweiten Fall mit separater Lyophilisation beträgt die Gesamtzeit ca. 20 bis 30 min. Die Proben sind dann für die Gelelektrophorese bereit. Im komplizierteren Fall zur Sequenzierung großer Anzahlen von DNA-Fragmenten müssen die vier Reaktoren räumlich andere Ausmaße besitzen. Die Grundfläche eines Reaktors beträgt bei 8 bis 15 zu sequenzierenden Fragmenten ca. 0,5 x 6 cm. Die vier (0,4 x 5,5 cm) großen Flächenträger mit aufgetragenen 8 bis 15 Fragmenten werden wiederum in die Reaktoren eingebracht, worauf die Schritte 1) und 2), selbstverständlich mit größeren Volumina an Reagentien, durchgeführt werden. Nach Ablassen der Reagentien der Modifizierungsreaktionen schließt sich ein Waschen (Operation 3) an. Danach werden die getrockneten Flächenträger automatisch in bestimmter vorgegebener Weise in die einzelnen, kleineren Stücke zerschnitten und dabei gleichzeitig jeweils in unterschiedliche Piperidin-Reaktoren mit Fritte und Ablaufventil (ca. 50 Reaktoren) eingebracht. Am besten gelingt dies mit vier beweglichen Modifizierungsreaktoren, die sich

nach Schritt 3) um 180° senken und sich dann am Reaktordeckel öffnen. Die Flächenträger werden dabei (durch vertikale Verschiebung) in vier bewegliche Schneidevorrichtungen gebracht. Durch Abschneiden kleiner Stücke von den Flächenträgern in den vier vertikal verschiebbaren Schneidevorrichtungen werden die 4 x 8 bis 15 = 32 bis 45 Piperidin-Reaktoren gezielt mit nur einem Fragment beschickt. Die Piperidin-Reaktionen laufen analog, wie bereits beschrieben, ab. Nach Beendigung der Reaktion werden die Piperidinlösungen mit den eluierten Fragmenten durch leichten Luftüberdruck in die mit den Reaktoren über PTFE-Schläuche verbundenen Eppendorf-Hütchen (ca. 50 Stück) übergeführt und anschließend lyophilisiert.

Beispiel 17

Sequenzierung unter Anwendung hoher Ionenstärken zur Elution der Nucleinsäurefragmente vom Träger

Bei Verwendung des beschriebenen Flächenträgers kann die Elution der Fragmente vor der Piperidin-Reaktion auch mit 1 bis 2 M Lösungen von $NH_4HCO_3$, $(NH_4)_2CO_3$, $NH_4Ac$, $Et_3NHAc$ u.a. erfolgen. Die Elution wird dadurch erreicht, daß die Flächenträger einzeln in Reaktionsgefäßen wie Eppendorf-Hütchen zweimal mit 50 µl der o. g. Salzlösungen bei 60 °C versetzt werden. Bei Oligonucleotiden werden die Eluate lyophilisiert, wobei sich die Salze bei wiederholter Lyophilisation mit wäßrigem Ethanol verflüchtigen lassen. Bei langen DNA-Fragmenten kann man die Salze auch durch Ethanolfällung entfernen. Danach erfolgt dann die übliche Piperidin-Reaktion in homogener Phase.

Beispiel 18

Die Grundausstattung der Vorrichtung zur Durchführung der Festphasensequenzierung gemäß der Erfindung besteht aus fünf Sequenzierungsblöcken 1, vier Deckeln 2, einem Probendosierer 3, einem Stanzer 5 und vier Flächenträgerhaltern 4 (vgl. Fig. 1-3). Der Sequenzierungsblock 1 weist einen quaderförmigen Grundblock 1.1 auf, in dem die Reaktionsgefäße 1.2 sowie eine Arretierung 1.3 eingelassen sind. Der Deckel 2 paßt genau auf den Sequenzierungsblock. Er hat eine auf der starren Abdeckung 2.2 befestigte elastische Abdeckung 2.1, die stopfenartige Erhöhungen 2.4 entsprechend der Zahl der Reaktionsgefäße 1.2 besitzt. Die Befestigungselemente 2.3 sind an den Ecken angeordnet. Das sind in der Regel zwei Schranken, die in dafür vorgesehene Gewindebohrungen im Sequenzierungsblock gedreht werden. Zum Flächenträgerhalter 4 gehören eine Grundplatte 4.1 mit der Vertiefung 4.2, eine Führungsplatte 4.3 und die Arretierung 4.5. Entsprechend der Zahl und dem Verteilungsmuster der Reaktionsgefäße 1.2 weisen Führungsplatte und Grundplatte Bohrungen 4.4 auf, die das Einführen der Stanzstifte 5.2 und der Kapillaren 3.2 gestatten. Der Durchmesser der Bohrungen 4.4 ist kleiner als der lichte Durchmesser der Reaktionsgefäße 1.2.

Der Probendosierer besteht aus dem Kapillarhalter 3.1 und den Kapillaren 3.2. Der Kapillarhalter 3.1, eine rechteckige Platte entsprechend der Fläche des Sequenzierungsblocks 1, hat ebensoviele Bohrungen und in der gleichen Anordnung, wie Reaktionsgefäße im Sequenzierungsblock vorhanden bzw. angeordnet sind. In den Bohrungen sind die Kapillaren 3.2 verschiebbar eingeführt, wobei die Mündung en 3.3 der Kapillaren 3.2 auf einer Ebene liegen, und der Abstand der Mündungen zum Kapillarhalter mindestens so groß ist, wie die Reaktionsgefäße 1.2 tief sind. Der Durchmesser der Kapillaren 3.2 ist etwas geringer als der Durchmesser der Bohrungen 4.4 des Flächenträgerhalters 4. Der Stanzer 5 hat die gleiche rechteckige Form wie der Sequenzierungsblock. Er besteht aus der Stempelplatte 5.1 und den dort eingelassenen Stanzstiften 5.2, die in ihrer Anzahl und in ihrer Verteilung der Anzahl und der Verteilung der Bohrungen 4.4 im Flächenträgerhalter entsprechen. Der Durchmesser der Stanzstifte ist so gewählt, daß sie genau in die Bohrungen 4.4 passen. Die Länge der Stanzstifte 5.2 ist mindestens so groß, wie die Grundplatte 4.1 und Führungsplatte 4.3 dick sind.

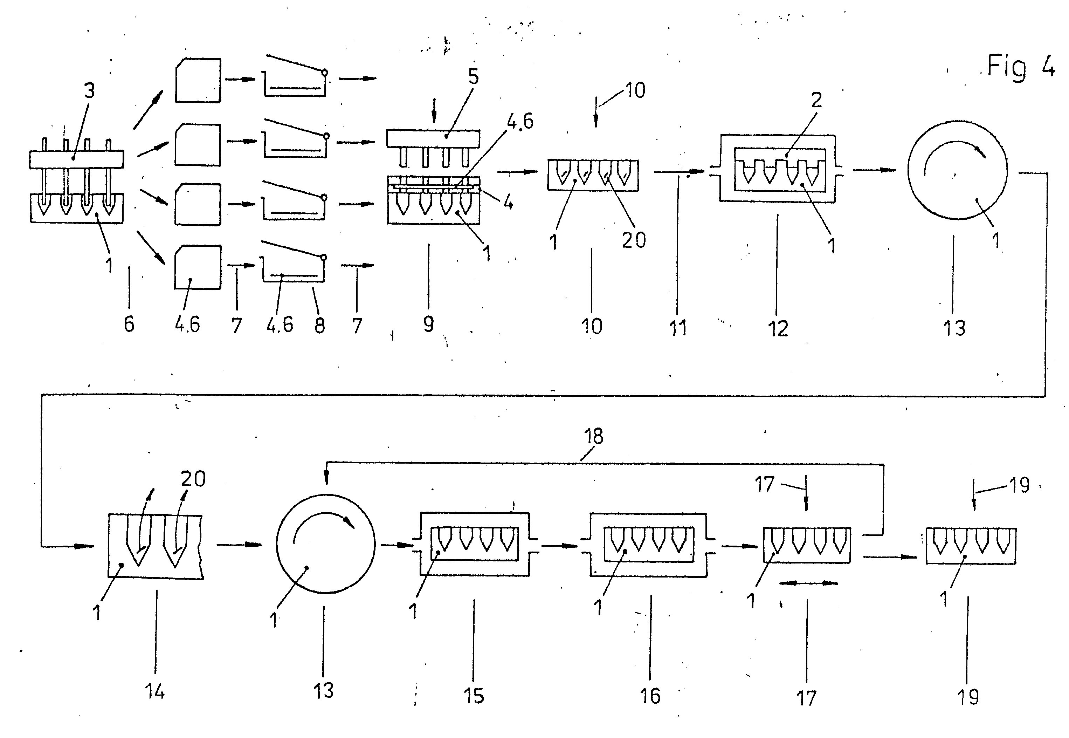

Zur Durchführung der Festphasensequenzierung werden die zu untersuchenden Proben in die Reaktionsgefäße 1.2 eines Sequenzierungsblocks 1 gefüllt. Mit Hilfe des Probendosierers 3 wird durch Eintauchen der Kapillaren in die wäßrige Lösung aufgrund der wirkenden Kapillarkräfte eine etwa gleichmäßige Füllung der Kapillaren erreicht (Fig. 2a). Anschließend wird der Probendosierer auf den saugfähigen Flächenträger 4.6 gedrückt, der unverrückbar in der Vertiefung 4.2 der Grundplatte 4.1 liegt. Dadurch wird Probenflüssigkeit auf den Träger übertragen. Der Flächenträger erhält ein Probenmuster, das dem Abordnungsmuster der Reaktionsgefäße entspricht. Der Abstand zwischen den aufgetragenen Proben bzw. zwischen den Reaktionsgefäßen ist so

gewählt, daß ein Ineinanderlaufen unmöglich ist (Fig. 1a). Die gleiche Prozedur erfolgt mit drei weiteren Flächenträgern, wobei die Anzahl der "Beimpfungen" pro Flächenträger gemäß dem oben beschriebenen Verfahren unterschiedlich ist, so daß nach Abschluß dieses Verfahrensschrittes vier "beimpfte" Flächenträger vorliegen, die nun den bekannten Modifizierungsreaktionen unterworfen werden. Danach erfolgt wiederum das Einlegen des Flächenträgers in den Flächenträgerhalter 4, und zwar in die dafür vorgesehene Vertiefung 4.2. Der Stanzer 5 wird mit seinen Stanzstiften 5.2 in die Bohrungen 4.4 eingeführt, und durch Herunterdrücken werden die Proben enthaltenden Bereiche des Trägers in die Reaktionsgefäße gestanzt (Fig. 3). Das gleiche erfolgt mit den weiteren drei beimpften Trägern, so daß vier Sequenzierungsblöcke mit den immobilisierten Proben in den Reaktionsgefäßen 1.2 vorliegen. Nun erfolgt die vom Verfahren vorgeschriebene Zugabe einer Piperidinlösung in die Reaktionsgefäße aller Sequenzierungsblöcke, worauf die Deckel 2 aufgesetzt und alle Blöcke gleichzeitig einer Temperaturbehandlung von 90 °C unterzogen werden. Dabei kommt es zum DNA-Strangbruch, und gleichzeitig erfolgt die Elution vom Träger. Die weiteren Verfahrensschritte wie Schütteln, Abtrennen der Trägerfragmente, Zentrifugieren, Abkühlen und Lyophilisieren, erfolgen in bzw. mit den Sequenzierungsblöcken (vgl. Fig. 4: 13 - 19). Die Weiterverarbeitung der Proben zur Trennung und Sichtbarmachung erfolgt durch Gelelektrophorese. Eine Verwechslung von Proben ist ausgeschlossen, da durch Anbringen von Markierungen nur eine Zuordnung der Elemente der Einrichtung wie Deckel 2, Probendosierer 3, Flächenträgerhalter 4 und Stanzer 5 zum Sequenzierungsblock möglich ist. Ein weiterer großer Vorteil ist die gleichzeitige Behandlung der Flächenträgerfragmente 20 bzw. der eluierten Proben bis zum Zeitpunkt des Auftragens auf die Gelelektrophorese ohne Entfernung aus dem Sequenzierungsblock. Dadurch wird eine beträchtliche Verringerung der Analysenzeit erzielt und durch Wahl der Anzahl der Reaktionsgefäße im Sequenzierungsblock ein hoher Probendurchsatz erreicht.

## Patentansprüche

1. Verfahren zur Festphasen-Sequenzierung von Nucleinsäurefragmenten,

   g e k e n n z e i c h n e t  d u r c h

   - Verwendung eines mechanisch stabilen Flächenträgers mit Anionenaustauscheigenschaften,
   - Immobilisierung der Fragmente an diesen Träger in an sich bekannter Weise,
   - anschließende chemische Modifizierung des immobilisierten Fragments oder mehrerer immobilisierter Fragmente gleichzeitig, gegebenenfalls mit Reagensüberschuß,
   - Waschen,
   - Umsetzung der sortierten Nucleinsäurefragmente einzeln mit Piperidin

     und
   - Elution mit chemischen, ionischen oder elektromagnetischen Mitteln.

2. Verfahren nach Anspruch 1, gekennzeichnet durch Verwendung eines mechanisch stabilen Flächenträgers mit Anionenaustauscheigenschaften, der durch Umsetzung geformter oder nicht geformter makromolekularer Massen, die aus

   - 5 bis 80 Vol.-% einer makromolekularen Verbindung,
   - 1 bis 80 Vol.-% einer makromolekularen Verbindung mit 4.6-Dihalogen-1.3.5-triazin-Gruppierungen,
   - 0,5 bis 50 Vol.-% eines 2,4,6-Trihalogen-1,3,5-

triazins,

- bis zu 20 Vol.-% Halogeniden und
- gegebenenfalls bis zu 5 Vol.-% puffernden Substanzen sowie
- gegebenenfalls aus einem flüssigen Dispergiermittel

bestehen,

mit Verbindungen der allgemeinen Formel I

$$\left[ X - R - \overset{\displaystyle R^1}{\underset{\displaystyle R^3}{\overset{|}{\underset{|}{N}}}} - R^2 \right]^{\oplus} \quad Y^{\ominus} \qquad (I)$$

erhältlich ist, in der bedeuten:

X $NH_2$, OH oder SH,

R $C_{1-10}$-Alkyl, aromatische Gruppen, photolytische Gruppen wie substituierte o-Nitrobenzylester- oder Methylphenancylestergruppen sowie heterocyclische Gruppen,

$R^1$, $R^2$, $R^3$ $C_{1-4}$-Alkyl oder H

und

Y Halogenidionen oder Hydroxylgruppen.

3. Verfahren nach Anspruch 1 oder 2, dadurch gekennzeichnet, daß die Verbindungen der allgemeinen Formel I intermediär durch Reaktion von Verbindungen der allgemeinen Formel II

$$\left[ Y - R - \overset{+}{N}H_3 \right]^{\oplus} \quad Y^{\ominus} \qquad (II)$$

mit tertiären Aminen der allgemeinen Formel III

$$\begin{array}{l} R^1 \\ | \\ N - R^2 \\ | \\ R^3 \end{array} \qquad (III),$$

worin R, $R^1$, $R^2$, $R^3$ und Y dasselbe wie in Anspruch 2 bedeuten, hergestellt werden.

4. Verfahren nach einem der Ansprüche 1 bis 3, dadurch gekennzeichnet, daß als geformte makromolekulare Massen mechanisch stabile Cellulose-Papiere, Vliese oder Gewebe eingesetzt werden.

5. Verfahren nach einem der Ansprüche 1 bis 4, dadurch gekennzeichnet, daß als ungeformte makromolekulare Massen kristalline, sphärische oder faserige Cellulose eingesetzt wird.

6. Verfahren nach einem der Ansprüche 1 bis 5, dadurch gekennzeichnet, daß die Immobilisierung von n Fragmenten auf mindestens 4 x n einzelnen Stücken des Flächenträgers vorgenommen wird.

7. Verfahren nach einem der Ansprüche 1 bis 6, dadurch gekennzeichnet, daß die Immobilisierung von n Fragmenten auf mindestens vier geometrisch größeren Flächenträgern vorgenommen wird, wobei auf jeden einzelnen Flächenträger n Fragmente aufgetragen werden, und nach den Modifizierungsreaktionen und den Waschoperationen jeder einzelne der vier Flächenträger in n einzelne Stücke zerschnitten und für die Piperidin-Reaktionen sortiert wird.

8. Verfahren nach einem der Ansprüche 1 bis 7, dadurch gekennzeichnet, daß zur Elution chemische Mittel wie primäre, sekundäre oder tertiäre Amine, insbesondere z. B. wäßrige Piperidin- oder wäßrige Anilinlösungen, gegebenenfalls bei höheren Temperaturen, verwendet werden.

9. Verfahren nach einem der Ansprüche 1 bis 7, dadurch gekennzeichnet, daß zur Elution Lösungen hoher Ionenstärke von Salzen wie $NH_4HCO_3$, $(NH_4)_2CO_3$, $Et_3NHCO_3$, $Et_3NHAc$, $NH_4Ac$, $K_2HPO_4$ und NaCl, gegebenenfalls bei höheren Temperaturen, verwendet werden.

10. Vorrichtung zur Festphasensequenzierung von Nucleinsäurefragmenten unter Verwendung von Flächenträgern, insbesondere nach dem Verfahren eines der Ansprüche 1 bis 9,

gekennzeichnet durch

vier Deckel (2), vier Flächenträgerhalter (4), einen Probendosierer (3) und einen Stanzer (5), die durch Markierungen, Führungen und Arretierungen in einer Orientierung eindeutig und unverwechselbar mit fünf Sequenzierungsblöcken (1) kombinierbar sind, wobei die Sequenzierungsblöcke aus einem Grundblock (1.1) mit darin eingelassenen Reaktionsgefäßen (1.2) und einer Arretierung (1.3) bestehen, die Deckel (2) aus einer elastischen Dichtung (2.1) mit stopfenartigen Erhöhungen (2.4) gemäß der Anzahl und dem Verteilungsmuster der Reaktionsgefäße (1.2),die mit einer starren Abdeckung (2.2) verbunden ist, und Befestigungselementen (2.3) bestehen, der Probendosierer (3) aus einem Kapillarhalter (3.1) mit den darin eingeführten Kapillaren (3.2) gemäß der Anzahl

und dem Verteilungsmuster der Reaktionsgefäße (1.2) besteht, wobei die Kapillarmündungen (3.3) auf einer planen Ebene liegen, und der Abstand der Ebene vom Kapillarhalter mindestens der Tiefe der Reaktionsgefäße (1.2) entspricht, die Flächenträgerhalter (4) aus einer Grundplatte (4.1) mit einer Vertiefung (4.2), deren Abmessungen in Höhe, Breite und Tiefe den Maßen des Flächenträgers (4.6) entsprechen, aus einer Führungsplatte (4.3), wobei Grundplatte (4.1) und Führungsplatte (4.3) genau übereinanderliegende Bohrungen (4.4) aufweisen, deren Anzahl und Verteilungsmuster denen der Reaktionsgefäße (1.2) entsprechen, und deren Durchmesser nur wenig größer als die Durchmesser der Kapillaren (3.2) sind, und einer Arretierung (4.5) bestehen, und der Stanzer (5) aus einer Stempelplatte (5.1) und Stanzstiften (5.2) besteht, deren Anzahl und Verteilungsmuster ebenfalls denen der Reaktionsgefäße (1.2) entsprechen, und die genau in die Bohrungen (4.4) passen.

11. Vorrichtung nach Anspruch 10, dadurch gekennzeichnet, daß die Reaktionsgefäße (1.2) eine zylindrische Form und/oder eine kegelige Form aufweisen.

12. Vorrichtung nach Anspruch 10 oder 11, dadurch gekennzeichnet, daß die Reaktionsgefäße (1.2) Eppendorf-Hütchen sind.

13. Vorrichtung nach einem der Ansprüche 10 bis 12, dadurch gekennzeichnet, daß die Reaktionsgefäße (1.2) ähnlich Mikrotiterplatten fest verbunden sind (1.4).

14. Vorrichtung nach einem der Ansprüche 10 bis 13, dadurch gekennzeichnet, daß die fünf Sequenzierungsblöcke unverwechselbar gekennzeichnet sind.

15. Vorrichtung nach einem der Ansprüche 10 bis 14, dadurch gekennzeichnet, daß vier Sequenzierungsblöcke zu einer Anordnung mit nur einer möglichen Orientierung zueinander zusammengefaßt sind.

16. Vorrichtung nach Anspruch 10 oder 11, dadurch gekennzeichnet, daß die Reaktionsgefäße (1.2) aus einer Folie tiefgezogen sind (1.4).

0 171 072

Fig 1

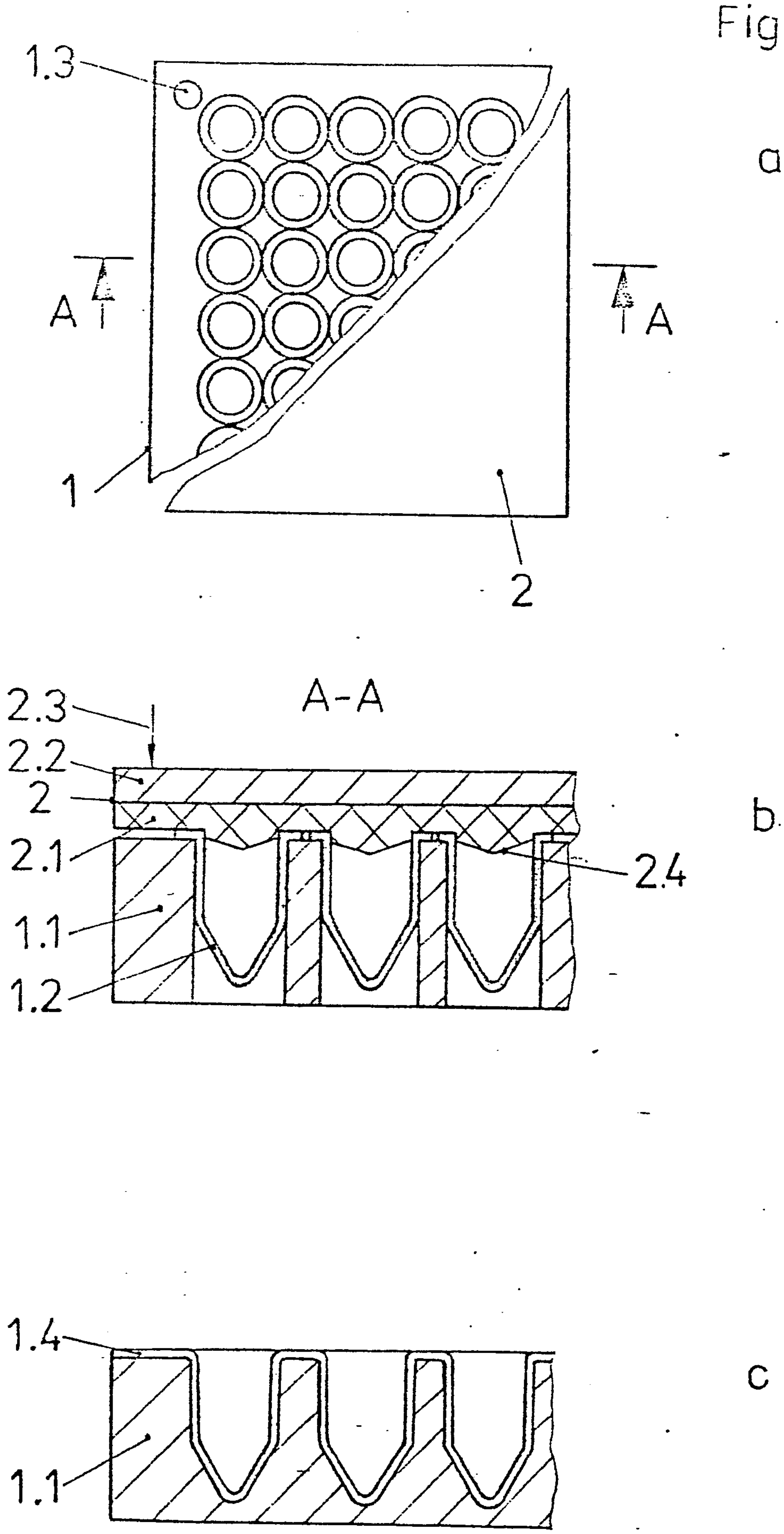

0 171 072

Fig 2

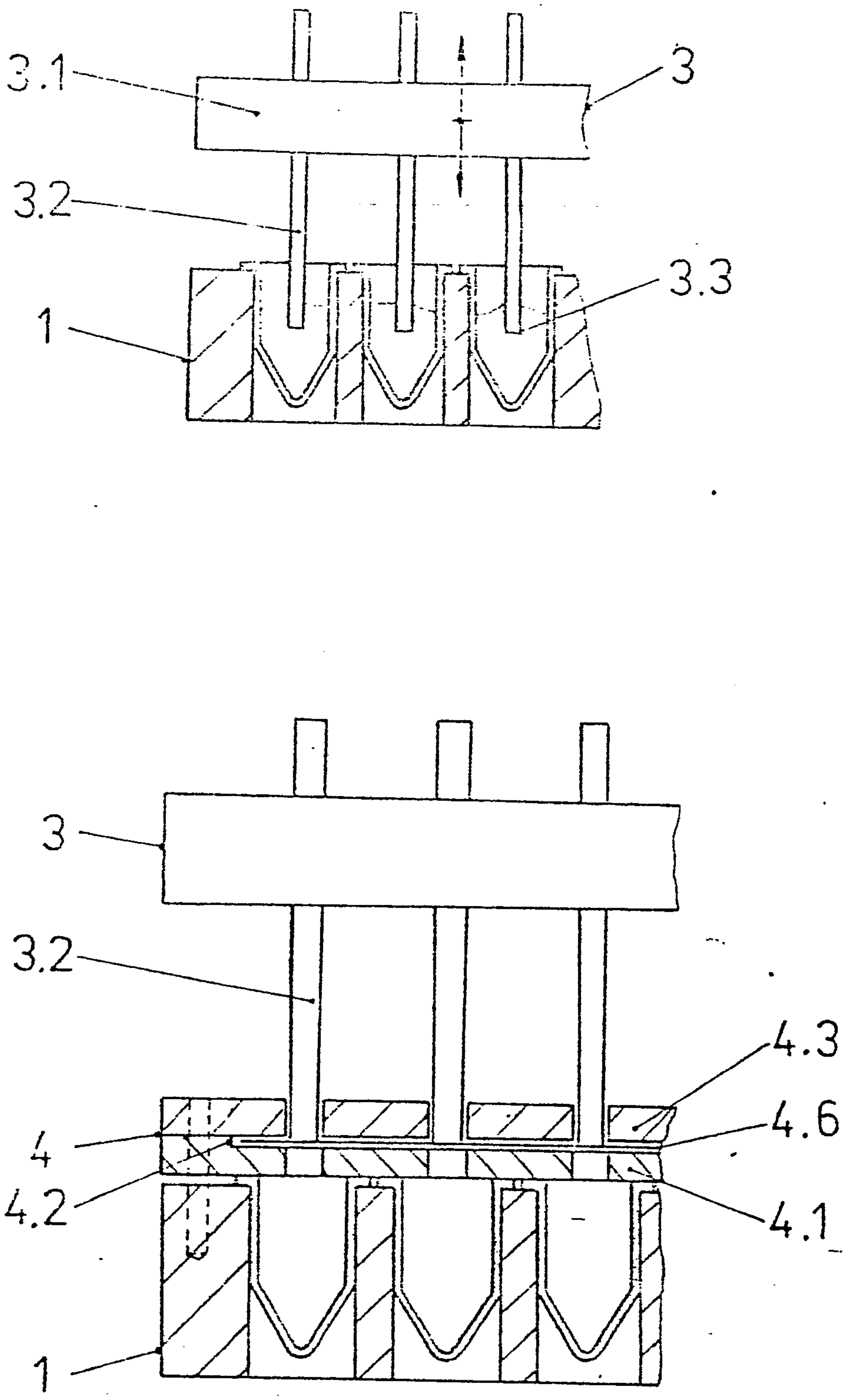


a

b

Fig 3

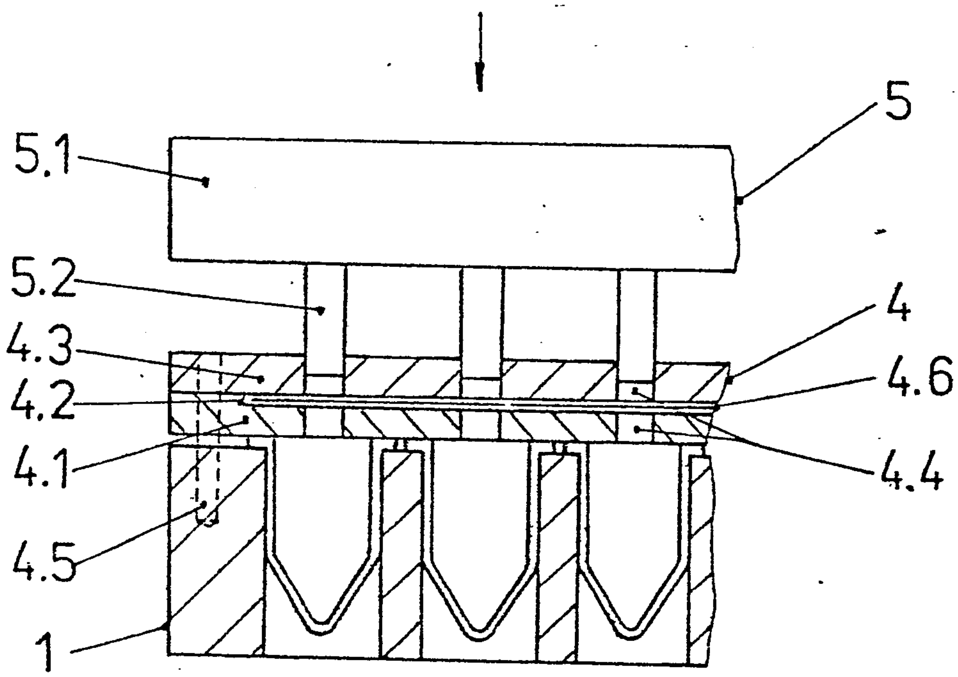

0 171 072

Fig 4